(19) **Europäisches Patentamt**

**European Patent Office**

**Office européen des brevets**

(11) **EP 1 256 351 A1**

(12) **EUROPEAN PATENT APPLICATION**
published in accordance with Art. 158(3) EPC

(43) Date of publication:
**13.11.2002 Bulletin 2002/46**

(51) Int Cl.⁷: **A61K 35/84**, A61P 35/00,
A61P 37/04, A61P 39/06,
A61P 43/00, A23L 1/28
// A23L1:22

(21) Application number: **00987787.9**

(22) Date of filing: **28.12.2000**

(86) International application number:
**PCT/JP00/09383**

(87) International publication number:
**WO 01/049308 (12.07.2001 Gazette 2001/28)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **05.01.2000 JP 2000000374**

(71) Applicant: **Kureha Chemical Industry Co., Ltd.
Tokyo 103-8552 (JP)**

(72) Inventor: **MATSUNAGA, Kenichi
Tokorozawa-shi, Saitama 359-1142 (JP)**

(74) Representative:
**Minderop, Ralph H., Dr. rer. nat. et al
Cohausz & Florack,
Patentanwälte,
Kanzlerstrasse 8a
40472 Düsseldorf (DE)**

(54) **NOVEL IMMUNE ENHANCING COMPOSITIONS**

(57) A novel immuno-enhancing composition or functional food, novel killer activity-inducing composition or functional food, novel tumor proliferation inhibitory composition or functional food, novel interleukin 12-inducing composition or functional food, novel TGF-β activity inhibitory composition or functional food, and novel active oxygen-capturing composition or functional food, comprising as an active ingredient a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin; and a novel adsorption fraction of a hot water extract of *Tricholoma matsutake* or an alkaline solution extract of *Tricholoma matsutake* by an anion exchange resin are disclosed.

EP 1 256 351 A1

**Description**

TECHNICAL FIELD

The present invention relates to a novel immuno-enhancing composition. The immuno-enhancing composition of the present invention may be administered as a medicament or in various forms, for example, eatable or drinkable products such as functional foods or health foods, or feeds. Further, the immuno-enhancing composition of the present invention may be administered in the form of an agent which is temporarily kept in the mouth but then spat out without retaining almost all of the components, for example, a dentifrice, a mouthwash agent, a chewing gum, or a collutorium, or in the form of an inhalation drawn in through the nose.

BACKGROUND ART

It is known that mushrooms contain many physiologically active substances. For example, Japanese Examined Patent Publication (Kokoku) No. 57-1230 and Japanese Patent No. 2767521 disclose various antitumor substances contained in *Tricholoma matsutake*. The above Japanese Examined Patent Publication (Kokoku) No. 57-1230 discloses that emitanine-5-A, emitanine-5-B, emitanine-5-C, and emitanine-5-D, which are separated and purified from a liquid extract obtained by extracting a broth of *Tricholoma matsutake* mycelia with hot water or a diluted alkaline solution, exhibits an activity of inhibiting a proliferation of sarcoma 180 cells. The above Japanese Patent No. 2767521 discloses that a protein with a molecular weight of 0.2 to 0.21 million (a molecular weight of a subunit = 0.1 to 0.11 million) which is separated and purified from an extract of *Tricholoma matsutake* fruit bodies with water exhibits an antitumor activity.
The inventor of the present invention compared and examined various physiological activities of extracts of many commercially available edible mushrooms with hot water. As a result, the inventor confirmed that an activity of inhibiting a proliferation of sarcoma 180 cells was detected in the extracts from many edible mushrooms including *Tricholoma matsutake*, whereas the inventor first found that a remarkably high activity of immuno-enhancement was detected only in the extract from *Tricholoma matsutake.* Further, the present inventor also found that an immuno-enhancing activity was exhibited not only in the hot water extract from *Tricholoma matsutake*, but also in an extract of *Tricholoma matsutake* with an alkaline solution, and further, in an adsorption fraction of the hot water extract of *Tricholoma matsutake* by an anion exchange resin or an adsorption fraction of the alkaline solution extract of *Tricholoma matsutake* by an anion exchange resin. It was not known that the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin exhibits an activity of immuno-enhancement as above. The present invention is based on the above findings.

DISCLOSURE OF INVENTION

The present invention relates to an adsorption fraction of a hot water extract of *Tricholoma matsutake* by an anion exchange resin or an adsorption fraction of an alkaline solution extract of *Tricholoma matsutake* by an anion exchange resin.
Further, the present invention relates to an immuno-enhancing composition, a killer activity-inducing composition (preferably, a composition for inducing a killer activity of an intestinal lymphocyte), a tumor proliferation inhibitory composition, an interleukin 12-inducing composition, a TGF-β activity inhibitory composition, or an active oxygen-capturing composition, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.
Further, the present invention relates to an immuno-enhancing functional food, a killer activity-inducing food (preferably a food for inducing a killer activity of an intestinal lymphocyte, a tumor proliferation inhibitory functional food, an interleukin 12-inducing functional food, a TGF-β activity inhibitory functional food, and an active oxygen-capturing functional food, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin with or without one or more food components.
Further, the present invention relates to a method for an immuno-enhancement, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.
Further, the present invention relates to a method for inducing a killer activity, preferably a killer activity of an intestinal lymphocyte, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

Further, the present invention relates to a method for inhibiting a tumor proliferation, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

Further, the present invention relates to a method for inducing an interleukin 12, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

Further, the present invention relates to a method for inhibiting a TGF-$\beta$ activity, comprising administering to a subject in need thereof a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin, in an amount effective therefor.

Further, the present invention relates to a method for capturing an active oxygen, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of an immuno-enhancing composition or a functional food.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or a functional food for inducing a killer activity, preferably a killer activity of an intestinal lymphocyte.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or a functional food for inhibiting a tumor proliferation.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or a functional food for inducing interleukin 12.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or a functional food for inhibiting a TGF-$\beta$ activity.

Further, the present invention relates to the use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or a functional food for capturing an active oxygen.

BRIEF DESCRIPTION OF DRAWINGS

Figure 1 illustrates a spectrum obtained by a $^1$H one-dimensional NMR measurement of the adsorption fraction D2.
Figure 2 illustrates a spectrum obtained by a $^{13}$C one-dimensional NMR measurement of the adsorption fraction D2.
Figure 3 illustrates a CD spectrum obtained by a circular dichroism analysis of the adsorption fraction D2.

BEST MODE FOR CARRYING OUT THE INVENTION

The present invention will be explained in detail hereinafter.

A novel fraction of the present invention which may be obtained by extracting *Tricholoma matsutake* with hot water, and adsorbing the extract by an anion exchange resin, exhibits an excellent immuno-enhancing activity despite the extraction with hot water. The hot water extract of *Tricholoma matsutake* also exhibits an excellent immuno-enhancing activity. Further, the alkaline solution extract of *Tricholoma matsutake* also exhibits an excellent immuno-enhancing activity. Furthermore, a novel fraction of the present invention, which may be obtained by extracting *Tricholoma matsutake* with an alkaline solution and adsorbing the extract by an anion exchange resin, exhibits an excellent immuno-enhancing activity.

Therefore, the immuno-enhancing composition of the present invention contains, as an active ingredient, (1) the hot water extract of *Tricholoma matsutake*, (2) the alkaline solution extract of *Tricholoma matsutake*, (3) the adsorption fraction of the hot water extract of *Tricholoma matsutake* by an anion exchange resin, or (4) the adsorption fraction of the alkaline solution extract of *Tricholoma matsutake* by an anion exchange resin, and further, a pharmaceutically or veterinarily acceptable ordinary carrier.

The adsorption fraction of the hot water extract of *Tricholoma matsutake* by an anion exchange resin, which is used as the active ingredient in the immuno-enhancing composition of the present invention, may be prepared by, for example, but by no means limited to, a process comprising steps of extracting *Tricholoma matsutake* with hot water (hereinafter referred to as a hot water extracting step), adsorbing the resulting extract by an anion exchange resin (hereinafter referred to as an anion exchange resin-adsorbing step), and then eluting the adsorption fraction with an appropriate solvent (hereinafter referred to as an eluting step).

The *Tricholoma matsutake* used in the hot water extracting step may be, for example, a fruit body or a mycelium of a naturally occurring *Tricholoma matsutake*, or a mycelium or a broth obtainable by culturing *Tricholoma matsutake.* An example of the *Tricholoma matsutake* used in the hot water extracting step may be, for example, the *Tricholoma matsutake* CM627-1 strain established and maintained in the Biomedical Research Laboratories, Kureha Chemical Industry Co. Ltd.

When the fruit bodies or mycelia are used as the above-mentioned *Tricholoma matsutake*, it is preferable to crush, grind, or pulverize them, to enhance the extraction efficiency.

A temperature of hot water used in the hot water extracting step is preferably 60 to 100 °C, more preferably 80 to 98 °C. It is preferable to carry out the hot water extracting step with stirring or shaking, so that an extraction efficiency is enhanced. An extracting time may vary with the form of *Tricholoma matsutake*, for example, a phase of fruit bodies, mycelia, or a broth, a treated form, such as a crushed, ground, or pulverized form, a temperature of hot water, or a treating condition with or without stirring or shrinking, but is for example, 1 to 6 hours, preferably 2 to 3 hours.

The resulting extract liquor obtained in the hot water extracting step may be used as it is, namely, in the state containing insolubles, in the subsequent anion exchange resin-adsorbing step. Before the anion exchange resin-adsorbing step, however, it is preferable to remove the insolubles, or to remove the insolubles and then low molecular weight fractions from the extract liquor. For example, the insolubles may be removed by centrifuging the hot water extract containing such insolubles, and the resulting supernatant may be used in the anion exchange resin-adsorbing step. Alternatively, after removing the insolubles by centrifuging the hot water extract containing such insolubles, and dialyzing the resulting supernatant to remove low molecular weight fractions, preferably fractions of low molecular weight substances having a molecular weight of 3500 or less, the resulting liquor may be used in the next anion exchange resin-adsorbing step.

In the anion exchange resin-adsorbing step, a known anion exchange resin, for example, diethylaminoethyl (DEAE) cellulose or triethylaminoethyl (TEAE) cellulose, may be used.

An eluting solution used in the eluting step may be appropriately selected in accordance with an anion exchange resin used in the eluting step, and for example, an aqueous solution of sodium chloride may be used.

A fraction eluted by the eluting step may be used as the active ingredient of the immuno-enhancing composition of the present invention as it is, i.e., without purification. However, the fraction eluted by the eluting step usually contains salts derived from the eluting solution, and therefore, it is preferable to dialyze the fraction and remove the salts.

For example, the adsorption fraction of the hot water extract of *Tricholoma matsutake* by an anion exchange resin, that is, one of the active ingredients of the immuno-enhancing composition of the present invention, has the following physicochemical properties, although it is understood that the fraction is not limited to those exhibiting the following physicochemical properties. The numerical values shown below are the physicochemical properties with respect to the adsorption fraction D2 prepared in Example 2, that is, an embodiment of the adsorption fraction of the hot water extract of *Tricholoma matsutake*, and determined in accordance with the methods disclosed in "Examination of Physicochemical Properties of the Adsorption Fraction D2" in Examples as mentioned below.

(1) Carbohydrate content: 13% in glucose equivalent
(2) Protein content: 86% in albumin equivalent
(3) Carbohydrate composition:
Mannose 47.7 µg/mg, galactose 32.43 µg/mg, glucose 25.09 µg/mg, arabinose 12.09 µg/mg, ribose 8.30 µg/mg, xylose 3.75 µg/mg, and rhamnose 0.44 µg/mg.
(4) Amino acid composition:
Aspartic acid and asparagine 14.12 mol%, threonine 6.39 mol%, serine 7.64 mol%, glutamic acid and glutamine 13/83 mol%, glycine 14.03 mol%, alanine 7.77 mol%, valine 5.36 mol%, 1/2-cystine 0.87 mol%, methionine 1.11 mol%, isoleucine 3.70 mol%, leucine 5.20 mol%, tyrosine 1.51 mol%, phenylalanine 2.28 mol%, lysine 4.05 mol%, histidine 1.67 mol%, arginine 2.52 mol%, and proline 7.93 mol%.

(5) Molecular weight distribution:

There are peaks widely ranging between approximately 45,000 to 1,000,000. There are five top peaks having estimated molecular weights of approximately 45,000, approximately 120,000, approximately 160,000, approximately 380,000, and 1,000,000 or more.

(6) Isoelectric points:

Three peaks are detected by isoelectric focusing. A main band is around 4.8 (4.5 to 5.2), and the other bands are around 7.6 (7.35 to 8.0) and around 9.2 (8.65 to 9.3).

(7) Ultraviolet spectroscopic analysis:

A peak was detected at 240 to 260 nm [the conditions for measurement are shown in item (7) of the "Examination of Physicochemical Properties of the Adsorption Fraction D2" as mentioned below].

(8) $^1$H one-dimensional NMR analysis:

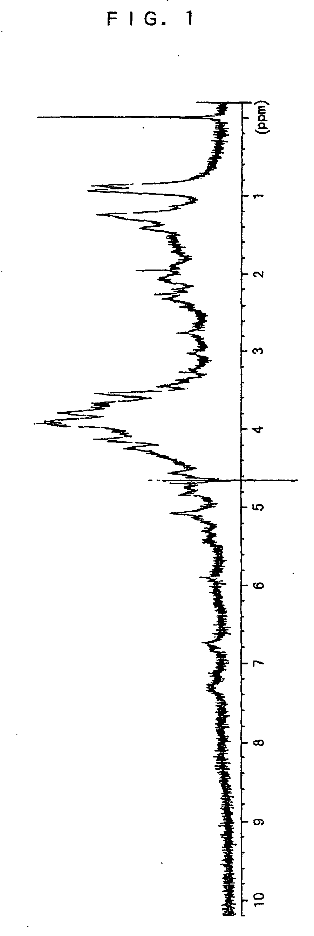

A spectrum as shown in Fig. 1 was obtained [the conditions for measurement are shown in item (8) of the "Examination of Physicochemical Properties of the Adsorption Fraction D2" as mentioned below].

(9) $^{13}$C one-dimensional NMR analysis:

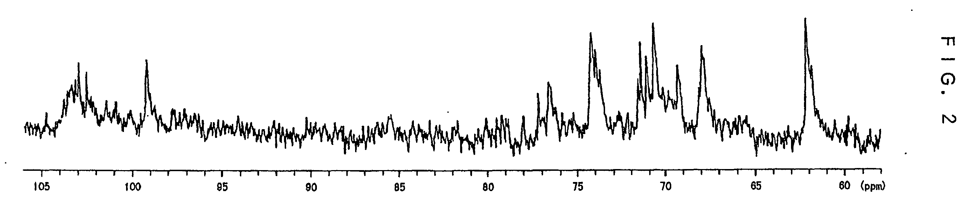

A spectrum as shown in Fig. 2 was obtained [the conditions for measurement are shown in item (8) of the "Examination of Physicochemical Properties of the Adsorption Fraction D2" as mentioned below].

(10) Circular dichroism analysis:

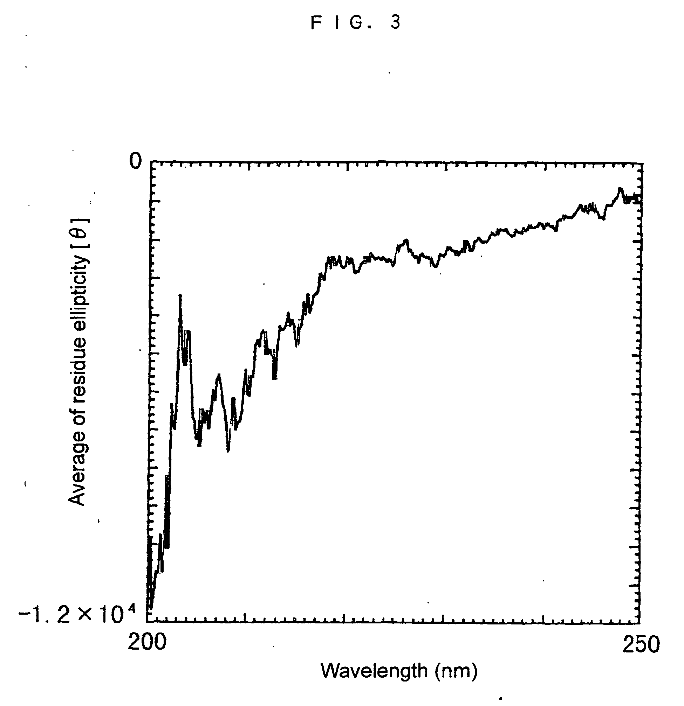

A spectrum as shown in Fig. 3 was obtained [the conditions for measurement are shown in item (9) of the "Examination of Physicochemical Properties of the Adsorption Fraction D2" as mentioned below]. The analysis of the secondary structure in accordance with the method of Chen et al. [Biochemistry, 11, 4120-4131 (1972)] revealed that α helix was 17%, β sheet was 18%, and an unordered structure was 65%.

The adsorption fraction of the alkaline solution extract of *Tricholoma matsukake* by an anion exchange resin, that is, one of the active ingredients of the immuno-enhancing composition of the present invention, may be prepared by, for example, but is by no means limited to, a method similar to that for preparing the adsorption fraction of the hot water extract of *Tricholoma matsutake* by an anion exchange resin as mentioned above. More particularly, the procedures of the above-mentioned method for preparing the adsorption fraction of the hot water extract of *Tricholoma matsutake* by an anion exchange resin can be repeated except that an alkaline solution is used instead of hot water, to prepare the adsorption fraction of the alkaline solution extract of *Tricholoma matsutake* by an anion exchange resin. For example, the adsorption fraction of the alkaline solution can be prepared by extracting *Tricholoma matsutake* with an alkaline solution (hereinafter referred to as an alkaline solution-extracting step), adsorbing the resulting extract liquor by an anion exchange resin (that is, the anion exchange resin-adsorbing step), and then eluting the adsorption fraction with an appropriate solvent (that is, the eluting step).

An alkaline solution used in the alkaline solution-extracting step may be, for example, but is by no means limited to, an aqueous solution of a hydroxide of an alkaline metal, such as sodium or potassium, particularly sodium hydroxide. A pH value of the alkaline solution is preferably 8 to 13, more preferably 9 to 12. The alkaline solution-extracting step is carried out preferably at 0 to 20 °C, more preferably at 0 to 15 °C. The resulting extract liquor of the alkaline solution-extracting step may be used in the subsequent anion exchange resin-adsorbing step, after neutralization.

The immuno-enhancing composition of the present invention can be administered to an animal, preferably a mammal, more preferably a human, in the form of a mixture of the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin, with a pharmaceutically or veterinarily acceptable ordinary carrier.

The active ingredient of the present invention, that is, the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin, exhibits an immuno-enhancing activity, for example, an activity to induce a killer activity, preferably an activity to induce a killer activity of an intestinal lymphocyte, an activity to inhibit a tumor proliferation, an activity to induce a cytokine, particularly interleukin 12, an activity to inhibit a TGF-β activity, or an activity to capture an active oxygen.

Therefore, the active ingredient of the present invention, that is, the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin, can be administered to a subject in need of an immuno-enhancement, for example, an induction of a killer activity, preferably an induction of a killer activity of an intestinal lymphocyte, an inhibition of a tumor proliferation, an induction of a cytokine, particularly interleukin 12, an inhibition of a TGF-β activity, or a capture of an active oxygen, with or without, but preferably with, a pharmaceutically or veterinarily acceptable ordinary carrier, in an amount effective therefor.

Further, the active ingredient of the present invention, that is, the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin, can be used in the manufacture of an immuno-enhancing composition or functional food, for example, a composition or functional food for inducing a killer activity, preferably a killer activity of an intestinal lymphocyte, a composition or functional food for inhibiting a tumor proliferation, a composition or functional food for inducing an interleukin 12, a composition or functional food for inhibiting a TGF-β activity, a composition or functional food for capturing an active oxygen.

The formulation of the immuno-enhancing composition of the present invention is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.

When the injections are prepared, for example, watersoluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the fraction as the active ingredient.

The immuno-enhancing composition of the present invention may be administered in the form of a sustained release preparation using sustained release polymers. For example, the immuno-enhancing composition of the present invention may be incorporated to a pellet made of ethylenevinyl acetate polymers, and the pellet may be surgically implanted in a tissue to be treated.

The immuno-enhancing composition of the present invention may contain the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin in an amount of, but is by no means limited to, 0.01 to 99% by weight, preferably 0.1 to 80% by weight.

A dose of the immuno-enhancing composition of the present invention is not particularly limited, but may be determined dependent upon the kind of disease, the age, sex, body weight, or symptoms of the subject, a method of administration, or the like. The immuno-enhancing composition of the present invention may be orally or parenterally administered.

The immuno-enhancing composition of the present invention may be administered as a medicament or in various forms, for example, eatable or drinkable products, such as functional foods or health foods, or feeds. Further, the immuno-enhancing composition of the present invention may be administered in the form of an agent which is temporarily kept in the mouth, but then spat out without retaining almost all of the components, for example, a dentifrice, a mouthwash agent, a chewing gum, or a collutorium, or in the form of an inhalation drawn in through the nose. For example, the hot water extract of *Tricholoma matsutake* or the alkaline solution extract of *Tricholoma matsutake*, or the adsorption fraction of the hot water extract of *Tricholoma matsutake* or the alkaline solution extract of *Tricholoma matsutake* by an anion exchange resin may be added to a desired food including a drink, a feed, a dentifrice, a mouthwash agent, a chewing gum, a collutorium, or the like as an additive, such as a food additive.

EXAMPLES

The present invention now will be further illustrated by, but is by no means limited to, the following Examples.

Example 1: Examination for biological activities of a hot water extracts of mushrooms

(1) Preparation of a hot water extracts of mushrooms

After 250 g of fruit bodies of commercially available *Tricholoma matsutake* harvested in Iwate Prefecture were lyophilized to remove water, the lyophilized fruit bodies were crushed to obtain 35 g of powder.

A portion (20 g) of the powder and 800 mL of purified water were charged in a 1-liter beaker, and an extraction treatment was performed in a water bath at 93 - 98 °C for 3 hours while stirring. After the extraction was completed, the whole was cooled to room temperature and centrifuged at 12,000 rpm for 20 minutes to obtain a supernatant.

To the remaining pellets, 500 mL of purified water was added, and the same procedures as above were performed. These procedures were repeated three times. Supernatants obtained by each procedure and the supernatant previously obtained were combined. The resulting mixture was put into a dialysis membrane with a fractioning molecular weight of 3500 (Spetra/Por 3 membrane; Spectrum, USA), and dialyzed in flowing tap water for 2 days. The inner part of dialyzate was concentrated by a rotary evaporator and lyophilized to obtain 1.12 g of powder.

Further, thirteen commercially available edible mushrooms were treated by the same process as above to obtain powders, respectively. The mushrooms and the yield (mass% with respect to a dried mushroom) are shown in Table 1.

Table 1

| No. | Mushrooms | Yield |
|-----|-----------|-------|
| 1 | *Tricholoma matsukake* | 5.6 |
| 2 | *Lentinus edodes* | 6.0 |
| 3 | *Flammulina velutipes* | 5.5 |
| 4 | *Pleurotus ostreatus* | 5.3 |
| 5 | *Grifola frondosa* | 4.9 |
| 6 | *Pholiota nameko* | 7.0 |
| 7 | *Pleurotus eryngii* | 5.8 |
| 8 | *Umbilicaria* | 4.6 |
| 9 | *Auriicularia auricula-judae* | 4.8 |
| 10 | *Agaricus blazei* | 7.3 |
| 11 | *Lyophyllum ulmarium* | 6.9 |
| 12 | *Grifola albicans* | 5.1 |
| 13 | *Agaricus bisporms* | 6.3 |
| 14 | *Porcini* | 5.5 |

(2) Examination for biological activities of hot water extracts of mushrooms

Powdery products obtained from edible mushroom extract liquors in Example 1(1) were orally administered to mice, and an influence producing an induction of a killer activity of an intestinal lymphocyte was examined. More particularly, biological activities were examined by taking cells of a mesenterium lymph node from a mouse to which tumor cells had been implanted at a cecal wall, and measuring a killer activity obtained when the tumor cells were re-stimulated in a test tube. The tumor cells used in this Example were mouse leukemic cells P815 and B7/P815 which were originally supplied from Dr. Mamoru Harada, Medical Institute of Bioregulation, Kyushu University, and maintained in an RPMI 1640 medium containing 10% bovine fetal serum which had been heated at 56 °C for 30 minutes, at Biomedical Research Laboratories, Kureha Chemical Industry Co. Ltd. Female DBA/2 mice were purchased from Japan SLC and used in experiments at 8 weeks-old after pre-breeding.

The mice were anesthetized by intraperitoneally administering 50 mg/kg of pentobarbital (Dainippon Pharmaceutical Co., Ltd.) and fixed. An abdomen was opened by scissors and tweezers, a cecum was taken out, B7/P815 cells ($1 \times 10^6$/50 μL) were implanted at a cecal wall, using 1 mL syringe equipped with a 1/8G dental needle (Hatajirushi Motoki Syringe Needle), and then the abdomen was closed by anatomical staplers. The mice which had recovered from anesthetization were put into a breeding cage, and bred under ordinary breeding conditions. From the day after the implantation of the tumor cells, each sample was orally administered at a dose of 500 mg/kg/day for 10 days in succession, using a probe for an oral administration. A group of mice contained 5 to 10 mice.

On the day after the last administration day, the mice were sacrificed. Then, a lymph node of a mesenterium was aseptically taken out, put on a sterilized dish containing a Hanks balanced salt solution, teased by scissors and tweezers, and passed through a mesh to prepare a single-cell suspension of lymphocyte cells. The cells were washed with an RPMI 1640 medium containing a 10% bovine fetal serum which had been heated three times at 56 °C for 30 minutes. Then, a concentration of cells was adjusted to $5 \times 10^6$/mL with an RPMI 1640 medium containing a 10% bovine fetal serum which had been heated at 56 °C for 30 minutes, $5 \times 10^{-5}$ mol/L of 2-mercaptoethanol, 20 mmol/L of 4-(2-hydroxyethyl)-1-piperazine ethanesulfonate, and 30 μg/mL of gentamicin and used as effector cells.

Stimulating cells were prepared as follows: P815 cells or B7/P815 cells were suspended in an RPMI 1640 medium containing a 10% bovine fetal serum which had been heated at 56 °C for 30 minutes, so that a concentration became $5 \times 10^6$/mL, and mitomycin C (Sigma) was added thereto so that a concentration thereof was 50 μg/mL. After a reaction was performed in a 5% carbon dioxide gas incubator for 30 minutes, the cells were washed with an RPMI 1640 medium containing a 10% bovine fetal serum which had been heated at 56 °C for 30 minutes three times, and a concentration of cells was adjusted to $1 \times 10^5$/mL.

A mixed lymphocyte tumor cell reaction was examined under the following conditions.

The effector cells (0.1 mL) and/or the stimulating cells (0.1 mL) were put on a 96-well culturing flat-bottomed microplate (Falcon 3072;Becton Dickinson Labware, USA), cultured in a 5% carbon dioxide gas incubator for 3 days, and recovered on a filter. When both the effector cells and the stimulating cells were put into the microplate, a ratio of the cells (the number of the effector cells/the number of the stimulating cells) was 12.5. In this examination system, the effector cells were functioned as lymphocytes in the mixed lymphocyte tumor cell reaction, and the stimulating cells were functioned as tumor cells in the mixed lymphocyte tumor cell reaction. Before 24 hours of completing the culturing, 37 kBq of $^3$H-thymidine (Amersham Japan) was added to each well of the plate. The harvested cells were thoroughly washed with 5% trichloroacetic acid, dried, and put into a vial for liquid. After adding a liquid scintillator, a radioactivity was measured by a liquid scintillation counter.

A stimulation index (S.I.) was calculated by an equation:

$$[S.I.]=(Bmix-Bs)/(Be-Bs)$$

wherein Bmix is a radioactivity (unit = Bq) of a group of the mixed culture of the effector cells and the stimulating cells, Bs is a radioactivity (unit = Bq) of a group of the single culture of the stimulating cells, and Be is a radioactivity (unit = Bq) of a group of the single culture of the effector cells.

A lymphocyte tumor cell mixed culture-induced cytotoxicity was examined under the following conditions.

The effector cells (1.0 mL) and the stimulating cells (1.0 mL) were put on a 24-well culturing microplate (Culture Clastar; Costar 3524; Corning Inc., USA) at a ratio of the cells (the number of the effector cells/the number of the stimulating cells) of 12.5, and cultured in a 5% carbon dioxide gas incubator for 3 days. After the culturing was completed, the cells were recovered and washed three times with an RPMI 1640 medium containing 10% bovine fetal serum which had been heated at 56 °C for 30 minutes, and the number of only the effector cells in the cell suspension was counted, using a microscope, so that a concentration of the effector cells was adjusted to $2.5 \times 10^6$/mL.

P815 cells were reacted with sodium chromate (Amersham Japan) at 37 °C for 20 minutes. Unreacted radioactive substances were removed by washing with an RPMI 1640 medium containing 10% bovine fetal serum which had been heated three times at 56 °C for 30 minutes, and a concentration of tumor cells labeled with radioactive chromium was adjusted to $5 \times 10^4$/mL.

0.1 mL of the effector cells or a double-diluted series thereof and 0.1 mL of tumor cells labeled with radioactive chromium were put into a test tube, and reacted in a 5% carbon dioxide gas incubator at 37 °C for 4 hours. After the reaction was completed, 1.5 mL of an RPMI 1640 medium containing a 10% bovine fetal serum, which had been heated at 56 °C for 30 minutes, was added to each test tube, and thoroughly mixed by a mixer. The whole was centrifuged at 12,000 rpm for 5 minutes at 4 °C to obtain a supernatant, and a radioactivity was measured by a gamma counter.

A specific lysis (S.L.; unit = %) was calculated by an equation:

$$[S.L.(\%)]=\{(B-Bf)/(Bmax-Bf)\} \times 100$$

wherein B is a radioactivity (unit = Bq) of a supernatant of an experimental group, Bf is a radioactivity (unit = Bq) of a supernatant of a spontaneously releasing group, and Bmax is a radioactivity (unit = Bq) of a supernatant of a maximum releasing group. The spontaneously releasing group means a group of culturing only the tumor cells labeled with radioactive chromium, and the maximum releasing group means a group of culturing tumor cells labeled with radioactive chromium treated with Triton.

The results of the mixed lymphocyte tumor cell reaction and the influence on the lymphocyte tumor cell mixed culture-induced cytotoxicity for the samples prepared in Example 1(1) are shown in Table 2, when the ratio of the number of the effector cells/the number of the stimulating cells was 12.5. A significant enhancement was observed in the sample from fruit body of *Tricholoma matsutake.* To control groups, 0.2 mL of purified water was orally administered. In Table 2, "*" means that there was a significant difference of $p<0.05$ on the basis of the control group.

Table 2

| No. | Origin of sample | Influence on mixed lymphocyte tumor cell reaction (% to the control group) | Lymphocyte tumor cell mixed culture-induced cytotoxicity (% to the control group) |
|---|---|---|---|
| 1 | *Tricholoma matsutake* | 132 * | 175 * |
| 2 | *Lentinus edodes* | 118 | 92 |
| 3 | *Flammulina velutipes* | 112 | 103 |
| 4 | *Pleurotus ostreatus* | 111 | 103 |
| 5 | *Grifola frondosa* | 108 | 118 |
| 6 | *Pholiota nameko* | 107 | 103 |
| 7 | *Pleurotus eryngii* | 120 | 112 |
| 8 | *Umbilicaria* | 101 | 120 |
| 9 | *Auriicularia auricula-judae* | 109 | 103 |
| 10 | *Agaricus blazei* | 106 | 113 |
| 11 | *Lyophyllum ulmarium* | 108 | 106 |
| 12 | *Grifola albicans* | 110 | 114 |
| 13 | *Agaricus bisporms* | 102 | 97 |
| 14 | *Porcini* | 116 | 103 |

Example 2: Preparation of a hot water extract of *Tricholoma matsutake* and a fraction thereof adsorbed by an anion exchange resin

After 500 mL-conical flasks (10 flasks) each containing 100 mL of sterile medium (3% glucose and 0.3% yeast extract, pH 6.0) were inoculated with mycelia of *Tricholoma matsutake* CM627-1 strain subcultured in the Biomedical Research Laboratories, Kureha Chemical Industry Co. Ltd., cultivation was performed in a shaking incubator (250 rpm) at 22 °C for 4 weeks. The resulting broth was homogenized, and extraction was performed in a water bath at 93 to 98 °C for 3 hours while stirring. After extraction, the whole was cooled to room temperature and centrifuged at 12,000 rpm for 20 minutes at 4 °C to obtain a supernatant.

To the remaining pellets, 500 mL of purified water was added, and the same procedures as above were performed. These procedures were repeated three times. All the resulting supernatants were combined. The resulting mixture was put into a dialysis membrane (Spetra/Por 3 membrane, fractioning molecular weight = 3500), and dialyzed in flowing tap water for 3 days. The inner part of dialyzate was concentrated by a rotary evaporator and lyophilized to obtain 3.9 g of powder (fraction D).

The powder (fraction D) was dissolved in 50 mmol/L tris-HCl buffer (pH 7.0), and the solution was applied to a diethylaminoethyl (DEAE)-cellulose column (diameter = 2 cm, height = 20 cm) which had been equilibrated with the buffer. The column was eluted by 100 mL of the buffer to obtain a non-adsorption fraction D1 (hereinafter sometimes referred to as "D1 fraction"). Then, 200 mL of a solution prepared by adding 1 mol/L sodium chloride into the buffer was applied to the column to elute adsorbed substances and obtain an adsorption fraction D2 (hereinafter sometimes referred to as "D2 fraction"). The obtained non-adsorption fraction and adsorption fraction were put into dialysis membranes (Spetra/Por 3 membrane, fractioning molecular weight = 3500), and dialyzed in purified water for 3 days, respectively. The inner parts of dialyzate were concentrated by a rotary evaporator and lyophilized to obtain 1.9 g of the non-adsorbed fraction D1 powder and 1.5 g of the adsorbed fraction D2 powder.

The immune activity of each faction was examined in the same manner as described in "Examination for biological activities of hot water extracts of mushrooms" (2). As shown in Table 3, the activity was observed in the adsorption fraction D2. In Table 3, "*" means that the significant difference ($p < 0.01$ with respect to the control group) was observed, and "**" means that the significant difference ($p < 0.05$ with respect to the control group) was observed. Further, the fraction D means a fraction before applying to the DEAE-cellulose column.

Table 3

| Test No. | Sample/Dose | Mixed lymphocyte tumor cell reaction (% to the control group) | Lymphocyte tumor cell mixed culture-induced cytotoxicity (% to the control group) |
|---|---|---|---|
| 1 | D / 250 mg/kg | 158 * | 153 * |
|  | D1/ 250 mg/kg | 103 | 109 |
| 2 | D2/ 250 mg/kg | 162 * | 171 * |
|  | D2/ 2.5 mg/kg | 106 | 110 |
|  | D2/ 25 mg/kg | 134 ** | 143 * |
|  | D2/ 250 mg/kg | 151 * | 167 * |

<u>Examination of Physicochemical Properties of the Adsorption Fraction D2</u>

Physicochemical properties of the D2 fraction were examined. Measuring methods and the results will be described below.

(1) Determination of carbohydrates

Carbohydrate content in the D2 fraction was determined by colorimetry using a phenol-sulfuric acid method. The content of carbohydrates in the D2 fraction was 13% in glucose equivalent.

(2) Determination of proteins

Protein content in the D2 fraction was determined by colorimetry using a copper-Folin method. The content of proteins in the D2 fraction was 86% in albumin equivalent.

(3) Analysis of the carbohydrate composition

Into a tube, 1.0 mg of the D2 fraction and 0.2 mL of 2 mol/L trifluoroacetic acid were charged, and hydrolyzed at 100 °C for 6 hours. The reaction mixture was dried under a reduced pressure by an evaporator to obtain a residue. The residue was dissolved in 500 µL of purified water, and further diluted to a double volume or a ten-fold volume with purified water. To 50 µL of this solution, 500 ng of heptose was added as an internal standard substance, and the solution was applied to a high performance liquid chromatograph LC-9A (Shimadzu) equipped with a column TSK-gel Sugar AXGLC-9A (15 cm x 4.6 mm ID; Tosoh) and a spectrophotometer RF-535 (Shimadzu) as a detector. The column temperature was 70 °C. The mobile phase was a 0.5 M potassium borate buffer (pH 8.7), and the flow rate thereof was 0.4 mL/min. For the conditions of post-column labeling, 1% arginine/3% boric acid was used as a reaction reagent, the flow rate was 0.5 mL/min., the reaction temperature was 150 °C, and the wavelengths for detection were EX 320 nm and EM 430 nm.

The carbohydrate composition was as follows: Mannose 47.7 µg/mg, galactose 32.43 µg/mg, glucose 25.09 µg/mg, arabinose 12.09 µg/mg, ribose 8.30 µg/mg, xylose 3.75 µg/mg, and rhamnose 0.44 µg/mg, in the order of descending content.

(4) Analysis of the amino acid composition

Into a tube, 1.0 mg of the D2 fraction and 0.1 mL of 6 mol/L hydrochloric acid were charged, and hydrolyzed at 110 °C for 22 hours. The reaction mixture was dried under a reduced pressure by an evaporator to obtain a residue. The residue was dissolved in 1.0 mL of purified water, and 50 µL of the solution was used for an amino acid analysis. The quantitative determination was performed by a ninhydrin colorimetry using an amino acid analyzer L-8500 (Hitachi) as equipment.

The amino acid composition was as follows:

Aspartic acid and asparagine 14.12 mol%, threonine 6.39 mol%, serine 7.64 mol%, glutamic acid and glutamine 13/83 mol%, glycine 14.03 mol%, alanine 7.77 mol%, valine 5.36 mol%, 1/2-cystine 0.87 mol%, methionine 1.11 mol%, isoleucine 3.70 mol%, leucine 5.20 mol%, tyrosine 1.51 mol%, phenylalanine 2.28 mol%, lysine 4.05 mol%, histidine 1.67 mol%, arginine 2.52 mol%, and proline 7.93 mol%.

(5) Measurement of the molecular weight distribution

After 1.0 mg of the D2 fraction was dissolved in 1.0 mL of purified water, the mixture was filtered out through a filter of 0.22 μm to obtain a filtrate. The filtrate was applied to a high performance liquid chromatograph LC-9A (Shimadzu) equipped with a column (Asahipak GS-620; 50 cm x 7.6 mm ID; Ashahi Kasei), a differential refractometer detector RID-6A (Shimadzu) and a ultraviolet detector SPD-6A (Shimadzu). The column temperature was room temperature. The mobile phase was purified water, and the flow rate thereof was 0.5 mL/min.

There were peaks widely ranging between approximately 45,000 to 1,000,000. There were five top peaks having estimated molecular weights of approximately 45,000, approximately 120,000, approximately 160,000, approximately 380,000, and 1,000,000 or more.

(6) Analysis of the isoelectric point

After 190 μg of the D2 fraction was dissolved in 20 μL of purified water, saccharose was added to a half of the solution so that the concentration thereof became approximately 40% (volume/volume), and then an electrophoresis was performed. The conditions of the electrophoresis were as follows:

Gel: IEF-PAGEmini (4%, pH 3-10; Tefco).
Buffer for electrophoresis: (cathode) 0.04 mol/L sodium hydroxide solution, (anode) 0.01 mol/L phosphate solution.
Conditions for electrophoresis: The electrophoresis was performed at 100 V for 30 minutes, then at 300 V for 20 minutes, and at 500 V for 40 minutes.
PI marker: Each band was 1.35 g (Pharmacia).

Three broad peaks were detected. A main band was around 4.8 (4.5 to 5.2), and the other bands were around 7.6 (7.35 to 8.0) and around 9.2 (8.65 to 9.3).

(7) Ultraviolet spectroscopic analysis (UV)

The D2 fraction was dissolved in purified water (concentration = 0.5 mg/10 mL) and a UV was measured. As a detector, 2500PC (Shimadzu) was used. A peak was detected at 240 to 260 nm.

(8) Nuclear magnetic resonance analysis (NMR)

The conditions for measurements were as follows.

(i) $^1$H one-dimensional NMR measurement

As a detector, UNITY INOVA-500 (Varian) was used. As a solvent, deuterated guanidium hydrochloride containing $D_2O$ solution was used. The concentration was 4 mol/L. DSS was used as an internal standard. The measurement was performed at 23 °C.

The resulting spectrum is shown in Figure 1. Around approximately 4.5 ppm to 5.0 ppm, which is a range characterizing a proton at 1-position of a sugar, there were observed peaks which were presumed to show α1-position of galactose or glucose. Further, peaks which were presumed to show β1-position of galactose or glucose were observed around 5.0 to 5.2 ppm.

(ii) $^{13}$C one-dimensional NMR measurement

The measurement was performed at the frequency of 150.8 MHz. The concentration was 20.3 mg/0.75 mL. The internal standard was a deuterated methanol (3% deuterated oxide solution, δ=49ppm). The temperature was 45 °C. Measurement of decoupling was performed under conditions of $^1$H complete decoupling.

The results are shown in Figure 2. It was assumed that a broad peak around 103 to 104 ppm showed a carbon at 1-position of galactose or glucose, and the carbon was glucoside-bonded in a β-type. Further, it was assumed that peaks around 102.96 ppm and 102.51 ppm showed α1-position and β1-position of mannose, respectively, and they were glucoside-bonded in a β-type.

Carbons at positions other than 1-position were analyzed by chemical shifts of peaks in 60 to 80 ppm. It was assumed that the carbon at β2-position of galactose or glucose, or the carbon at 2(α, β)-position of mannose was shifted, by comparing chemical shifts of each monosaccharide. Further, in a carbons of galactose or glucose, there was observed no carbon whose chemical shift was changed in comparison with monosaccharides. Therefore, it was assumed that the carbon at 6-position was shifted and superimposed on the signal (67.8 ppm) of 4-position of mannose, because a strength of this peak was large. The above analysis showed that 2($\beta$)-position and 6($\alpha$)-position of galactose or glucose, or 2($\alpha$, $\beta$)-position of mannose is assumed to participate in a bonding at the position other than 1-position of sugars. However, accuracy of the above analysis was not high, because the resulting peaks were generally broad, and a S/N ratio was bad.

(10) Circular dichroism analysis (CD)

The conditions for measurement were as follows:

As a detector, JASCOJ-500A was used. As a solvent, water was used. The concentration of proteins was 0.125 mg/mL. The wavelength area was 200 to 250 nm. The cell length was 1 mm. The temperature was room temperature (24 °C). The number of accumulation was 8. The measurement was performed under the above conditions.

The resulting CD spectrum is shown in Figure 3. The CD value (vertical axis) is an average of the residual ellipticity [$\theta$]. The unit of [$\theta$] is deg·cm$^2$·decimol$^{-1}$. As an average molecular weight of residues for conversion into [$\theta$], 103.45 was used. It was determined from an amino acid analysis. The analysis of the secondary structure according to a method of Chen et al. showed that $\alpha$-helix was 17%, $\beta$-sheet was 18%, and unordered structure was 65%.

Example for evaluation of biological activities of the adsorption fraction D2

(1) Activity of the adsorption fraction D2 for inhibiting tumor (sarcoma 180) proliferation

As an animal subject, female ICR mice (CLEA Japan, Inc.) were used. As a tumor, sarcoma 180 cells maintained in the peritonium of a female ICR mouse in Biomedical Research Laboratories, Kureha Chemical Industry Co. Ltd., were used. More particularly, sarcoma 180 cells ($1\times10^6$) were transplanted at an axilla of 5-week-old female ICR mice (one group consisting of 10 mice). From the day after the transplantation, a predetermined amount (1.0 mg/kg, 10 mg/kg, or 50 mg/kg) of the adsorption fraction D2 obtained in Example 2 was intraperitoneally administered every other day and 10 times in total. On the 25th day after the transplantation, mice were sacrificed, tumor nodes were taken, and the weights were measured. Physiological saline was administered to the mice of the control group.

The ratio of proliferation inhibition (unit = %) was calculated by the following equation:

$$[\text{Ratio of proliferation inhibition (\%)}] = \{(Wc-W)/Wc\}\times100$$

wherein W is an average weight (unit = g) of the node taken from the group treated with the sample, and Wc is an average weight (unit = g) of the node taken from the group treated with the physiological saline.

The results are shown in Table 4. In Table 4, "*" means that the significant difference ($p<0.05$) was observed. As apparent from Table 4, proliferation was significantly inhibited by administering the D2 fraction.

Table 4

| No. | Rate of proliferation Sample/Dose | inhibition (%) |
|-----|-----------------------------------|----------------|
| 1 | D2 fraction/ 1.0 mg/kg | 65% * |
| 2 | D2 fraction/ 10 mg/kg | 76% * |
| 3 | D2 fraction/ 50 mg/kg | 46% |

(2) Cytokine inducing activity of the adsorption fraction D2

After 8-week-old female DBA/2 mice were sacrificed, the blood was removed, and mesenteric lymph nodes were aseptically taken and transferred to a sterile petri dish containing a Hanks' balanced salt solution. The lymph nodes were teased with scissors and tweezers, and passed through a mesh to prepare a suspension containing single lymphocyte cells. The cells were washed three times with a RPMI 1640 medium containing a 10% bovine fetal serum which had been heated at 56 °C for 30 minutes, and the cell suspension was adjusted to $2 \times 10^6$ cells/mL. The adsorption fraction D2 obtained in Example 2 was dissolved in the medium. The solution was sterilized through a filter to prepare a sample solution (250 µg/mL). Into the wells of a 96-well flat bottom microplate for cell culture (Falcon 3072; Becton Dickinson Labware, USA), 0.1 mL of the cell suspension and 0.1 mL of the sample solution were placed and cultured in a 5% $CO_2$ incubator at 37 °C for 18 hours. After the culture, each supernatant was separated by centrifugation. The total content of interleukin 12 in each culture supernatant was measured using a commercially available measuring kit (Intertest 12X; Genzyme, USA). The results are shown in Table 5. As apparent from Table 5, the D2 fraction exhibited the interleukin 12-inducing activity.

Table 5

| No. | Sample | Total content of interleukin 12 (pg/mL) |
|---|---|---|
| 1 | medium control | 0 (below a detectable limit) |
| 2 | D2 fraction (125 µg/mL) | 69 |

(3) Immunosuppressant TGF-β binding activity of the adsorption fraction D2

In a polypropylene tube (multi-siliconized tube, Safe Seal Microcentrifuge Tube; Funakoshi) which slightly adsorbed proteins, a commercially available preparation of human recombinant Transforming Growth Factor-$\beta_1$ (TGF-$\beta_1$; Funakoshi) was dissolved in a phosphate buffer (pH 7.2) containing 2% albumin, and the solution was adjusted to 100 ng/mL. Further, the adsorption fraction D2 prepared in Example 2 was dissolved in a phosphate buffer containing 2% albumin, and the solution was adjusted to 2 mg/mL. Into a tube which slightly adsorbed proteins, 0.5 mL of the TGF-$\beta_1$ solution and 0.5 mL of the D2 fraction solution were charged, and the reaction was performed at 22 °C for 3 hours. After the reaction was completed, the content of TGF-$\beta_1$ in the reaction solution was measured using a commercially available measuring kit (Quantikine Human TGF $\beta_1$ ELISA Kit; Funakoshi).

The binding rate (unit = %) was calculated by the following equation:

$$[\text{Binding ratio (\%)}] = \{(Tc\text{-}T)/Tc\} \times 100$$

wherein T is the found value (unit = ng/mL) of TGF-$\beta_1$ in the group wherein the D2 fraction was administered, and Tc is the found value (unit = ng/mL) of TGF-$\beta_1$ in the group wherein the phosphate buffer containing 2% albumin was administered.

The results are shown in Table 6. The binding of the D2 fraction and TGF-$\beta_1$ was observed in the group wherein the D2 fraction was administered.

Table 6

| No. | Sample | Binding rate % |
|---|---|---|
| 1 | Administration of phosphate buffer containing 2% albumin (Control) | 0 |
| 2 | D2 fraction (1 mg/mL) | 21 |

(4) Active oxygen-capturing activity of the adsorption fraction D2

After 1000 µL of 3.0 mol/L hypoxanthine solution, 5 µL of 2 U/mL xanthine oxidase (Boehringer Mannheim) solution, 500 µL of 8 mg/mL D2 fraction, and 495 µL of RPMI 1640 medium were charged into a test tube, 120 µL of 270 µmol/L 2-methyl-6-phenyl-3,7-dihydroimidazo-[1,2-α]pyrazine-3-on (CLA-phenyl; Tokyo Kasei) solution was further added. Further, the test tube containing the mixture was set into a chemiluminescence meter (Aloka) wherein the temperature of a reaction chamber was maintained at 37 °C. The reaction was performed for 30 minutes, and an amount of generated chemiluminescence was measured with time.

The capturing activity (unit = %) was calculated by the following equation:

$$[\text{Capturing activity (\%)}] = \{(Cc\text{-}C)/Cc\} \times 100$$

wherein C is an amount of chemiluminescence in the group wherein the D2 fraction was administered, and Cc is an amount of chemiluminescence in the group (control group) wherein a phosphate buffer was administered. The results are shown in Table 7. As apparent from Table 7, the D2 fraction exhibited the active oxygen-capturing activity.

Table 7

| No. | Sample | Capturing activity % |
|---|---|---|
| 1 | Phosphate buffer (pH 7.2) | 0 |
| 2 | D2 fraction (1 mg/mL) | 96 |

(5) Anti-tumor activity of the adsorption fraction D2

A Swiss albino 3T3 strain subcultured in 50 mL-flasks for cell culture (3014; Becton-Dickinson) in the Biomedical Research Laboratories, Kureha Chemical Industry Co. Ltd., and an SV40-transformed 3T3 cell strain [purchased from the Laboratory Products Department of Dainippon Pharmaceutical Co., Ltd., Osaka] were treated with 0.125% trypsin solution for several minutes, and released from the wall of each flask. After washing, cells were suspended in DMEM (Dulbecco's Modification of Eagle's Medium) containing a 10% bovine fetal serum which had been heated at 56 °C for 30 minutes, and adjusted to $2 \times 10^3$ cells/mL. Into each well of a 96-well flat bottom microtestplate for cell culture (3072; Becton Dickinson), 0.1 mL of the cell suspension was poured.

After incubation in a 5% $CO_2$ incubator at 37 °C for 24 hours, a predetermined amount of the adsorption fraction D2 prepared in Example 2 or 0.1 mL of the medium was added and further incubated for 24 hours. Before 6 hours of completing the cultivation, 1 µCi/well of $^3$H-thymidine (Amersham International plc) was added. After the cultivation was completed, cells were harvested on a filter paper, thoroughly washed with 5% trichloroacetic acid, and dried. Then, a radioactivity taken up to the cells was measured by a liquid scintillation counter. A 50% lethal dose ($LD_{50}$) of the adsorption fraction D2 was about 50 µg/mL in the SV40-transformed 3T3 cell strain, and about 40 µg/mL in the Swiss albino 3T3 strain. The results of the $LD_{50}$ measured according to a MTT method were similar to the above results by the $^3$H-thymidine method.

Example 3: Preparation of an alkaline solution extract of *Tricholoma matsutake* and an adsorption fraction thereof by an anion exchange resin

The broth of mycelia of *Tricholoma matsutake* CM627-1 strain was treated in a homogenizer. The broth was prepared in the similar manner as described in Example 2. A sodium hydroxide solution was added to the whole so that the concentration of the whole became 0.1 mol/L. Extraction was performed at 22 °C for 1 hour while stirring. After extraction, the whole was centrifuged (12,000 rpm, 20 minutes, 4 °C) to obtain a supernatant.

To the remaining pellets, a 0.3 mol/L sodium hydroxide solution was added. The same procedures as above were performed to obtain a supernatant. Further, a 0.5 mol/L sodium hydroxide solution was added to the pellets, and the same procedures as above were performed to obtain a supernatant. All of the supernatants were combined, and the pH of the supernatant was adjusted to 7.0 by adding 1 mol/L HCl thereto. The whole was put into a dialysis membrane (Spetra/Por 3 membrane, fractioning molecular weight = 3500), and dialyzed in flowing tap water for 3 days. The inner part of dialyzate was concentrated by a rotary evaporator and lyophilized to obtain 4.3 g of powder (hereinafter referred to as a fraction A).

The powder (the fraction A) was dissolved in a 50 mmol/L tris-HCl buffer (pH 7.0), and the solution was applied to a diethylaminoethyl (DEAE)-cellulose column (diameter = 2 cm, height = 20 cm) which had been equilibrated with the buffer. The column was eluted by 100 mL of the buffer to obtain a non-adsorption fraction A1 (hereinafter sometimes referred to as an "A1 fraction"). Then, 200 mL of a solution prepared by adding 1 mol/L sodium chloride into the buffer was applied to the column to elute adsorbed substances and obtain an adsorption fraction A2 (hereinafter sometimes referred to as an "A2 fraction"). The resulting non-adsorption fraction and adsorption fraction were put into dialysis membranes (Spetra/Por 3 membrane, fractioning molecular weight = 3500), and dialyzed in purified water for 3 days, respectively. The inner parts of dialyzate were concentrated by a rotary evaporator and lyophilized to obtain the non-adsorption fraction A1 powder and the adsorption fraction A2 powder.

Example for evaluation of biological activities of the fraction A

The activity for enhancing a proliferation of T cells was evaluated in the similar manner as described in "Examination for biological activities of a hot water extracts of mushrooms" (2). The activity in the group wherein the fraction A was administered (dose = 250 mg/kg) was 130% (% on the basis of the value of the control group) in the mixed lymphocyte tumor cell reaction, and 131% (% on the basis of the value of the control group) in the lymphocyte tumor cell mixed culture-induced cytotoxicity.

Further, the cytokine inducing activity was evaluated in a similar manner as that described in "Example for evaluation of biological activities of the adsorption fraction D2" (2). The total amount of interleukin 12 in the control group was less than a detectable limit, but the total amount of interleukin 12 in the group wherein the fraction A was administered was 69 pg/mL.

Furthermore, the TGF-β binding activity was evaluated in a similar manner as that described in "Example for evaluation of biological activities of the adsorption fraction D2" (3). The binding activity of the fraction A was 26%.

INDUSTRIAL APPLICABILITY

According to the immuno-enhancing composition of the present invention, an immune activity can be enhanced.

As above, the present invention is explained with reference to particular embodiments, but modifications and improvements obvious to those skilled in the art are included in the scope of the present invention.

**Claims**

1.  An adsorption fraction of a hot water extract of *Tricholoma matsutake* or an alkaline solution extract of *Tricholoma matsutake* by an anion exchange resin.

2.  An immuno-enhancing composition comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

3.  An immuno-enhancing functional food comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, alone or optionally with one or more food components.

4.  A method for an immuno-enhancement, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

5.  Use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of an immuno-enhancing composition or functional food.

6.  A composition for inducing a killer activity, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

7.  A functional food for inducing a killer activity, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, alone or optionally with one or more food components.

8.  A method for inducing a killer activity, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

9.  Use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or functional food for inducing a killer activity.

10.  A composition for inhibiting a tumor proliferation, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

11.  A functional food for inhibiting a tumor proliferation, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, alone or optionally with one or more food components.

12. A method for inhibiting a tumor proliferation, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

13. Use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or functional food for inhibiting a tumor proliferation.

14. A composition for inducing an interleukin 12, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

15. A functional food for inducing an interleukin 12, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, alone or optionally with one or more food components.

16. A method for inducing an interleukin 12, comprising administering to a subject in need thereof, a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

17. Use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or functional food for inducing an interleukin 12.

18. A composition for inhibiting a TGF-β activity, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

19. A functional food for inhibiting a TGF-β activity, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma* matsutake by an anion exchange resin, alone or optionally with one or more food components.

20. A method for inhibiting a TGF-β activity, comprising administering to a subject in need thereof a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in an amount effective therefor.

21. Use of a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, in the manufacture of a composition or functional food for inhibiting a TGF-β activity.

22. A composition for capturing an active oxygen, comprising a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin, and a pharmaceutically acceptable carrier.

**23.** A functional food for capturing an active oxygen, comprising a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin, alone or optionally with one or more food components.

**24.** A method for capturing an active oxygen, comprising administering to a subject in need thereof a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin, in an amount effective therefor.

**25.** Use of a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin, in the manufacture of a composition or functional food for capturing an active oxygen.

F I G. 1

F I G. 2

F I G. 3

**EP 1 256 351 A1**

# INTERNATIONAL SEARCH REPORT

| International application No. |
| --- |
| PCT/JP00/09383 |

A. CLASSIFICATION OF SUBJECT MATTER
Int.Cl$^7$  A61K35/84, A61P35/00, 37/04, 39/06, 43/00, A23L1/28 // A23L1/22

According to International Patent Classification (IPC) or to both national classification and IPC

B. FIELDS SEARCHED

Minimum documentation searched (classification system followed by classification symbols)
Int.Cl$^7$  A61K35/84, A61P35/00, 37/04, 39/06, 43/00, A23L1/28 // A23L1/22

Documentation searched other than minimum documentation to the extent that such documents are included in the fields searched

Electronic data base consulted during the international search (name of data base and, where practicable, search terms used)
CA (STN),BIOSIS (STN),MEDLINE (STN),EMBASE (STN)

C. DOCUMENTS CONSIDERED TO BE RELEVANT

| Category* | Citation of document, with indication, where appropriate, of the relevant passages | Relevant to claim No. |
| --- | --- | --- |
| X | GB, 1279104, A (Ajinomoto Co., Inc.), 28 June, 1972 (28.06.72) & FR, 2020160, A  & CA, 968287, A & CH, 529173, A | 1-3,5,10,11 13 |
| X | JP, 10-287584, A (Biken K.K.), 27 October, 1998 (27.10.98) (Family: none) page 2; Par. No. [003] | 1-3,5-7,9-11 13 |
| X | JP, 10-147534, A (Akikuni YAKIDA), 02 June, 1998 (02.06.98), page 4; Par. No. [0023], Par. No. [0025], & CN, 11853199, A | 1-3,5-7,9-11 13-15,17 |
| X | US, 5302699, A (Norinsuisansho Shokuhin Sogo, Japan), 12 April, 1994 (12.04.94) & JP, 6-80699, A | 1,10,11,13 |
| X | JP, 6-65575, A (Tanabe Seiyaku Co., Ltd.), 08 March, 1994 (08.03.94)(Family: none) | 1,22,23,25 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 03 April, 2001 (03.04.01) | Date of mailing of the international search report 17 April, 2001 (17.04.01) |
| --- | --- |
| Name and mailing address of the ISA/ Japanese Patent Office | Authorized officer |
| Facsimile No. | Telephone No. |

Form PCT/ISA/210 (second sheet) (July 1992)

21

**INTERNATIONAL SEARCH REPORT**

| International application No. |
| --- |
| PCT/JP00/09383 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | page 2; Par. No. [007]<br><br>Spec.  Publ. - R. Soc. Chem. (1993), 123 (Food and Cancer Prevention: Chemical and Biological Aspects), pages 327 to 330 "Anti-tumorigenic and immunoactive protein and peptide factor in foodstuffs. (I). Anti-tumorigenic protein from Tricholoma matsutake" Y. KAWAMURA and M. ISHIKAWA | 1-3,10,11,13 |

Form PCT/ISA/210 (continuation of second sheet) (July 1992)

## INTERNATIONAL SEARCH REPORT

| International application No. |
| --- |
| PCT/JP00/09383 |

**Box I    Observations where certain claims were found unsearchable (Continuation of item 1 of first sheet)**

This international search report has not been established in respect of certain claims under Article 17(2)(a) for the following reasons:

1. ☒ Claims Nos.: 4,8,12,16,20,24
   because they relate to subject matter not required to be searched by this Authority, namely:

   These claims pertain to methods for treatment of the human body by therapy (Rule 39.1(iv) of the Regulations under the PCT).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1992)